# EUROPEAN PATENT SPECIFICATION

(11) **EP 0 868 429 B1**
(45) Date of publication and mention of the grant of the patent: **16.10.2002**
(21) Application number: 96942326.8
(22) Date of filing: 04.12.1996
(51) Int. Cl.: C07K 5/062, C07K 5/083, C07K 5/103, C07K 5/078, C07K 5/117, A61K 38/05, A61K 38/06, A61K 38/07

(54) **OLIGOPEPTIDE COMPOUNDS CONTAINING D-2-ALKYLTRYPTOPHAN CAPABLE OF PROMOTING THE RELEASE OF GROWTH HORMONE**
D-2-ALKYLTRYPTOPHAN-ENTHALTENDE OLIGOPEPTIDVERBINDUNGEN, DIE DIE FREISETZUNG VON WACHSTUMSHORMON FÖRDERN
COMPOSES OLIGOPEPTIDIQUES CONTENANT UN D-2-ALKYLTRYPTOPHANE CAPABLES DE FAVORISER LA LIBERATION DE L'HORMONE DE CROISSANCE

(30) Priority: 20.12.1995 IT MI952681
(43) Date of publication of application: 07.10.1998
(73) Proprietor: Ardana Bioscience Limited, Edinburgh EH2 3NS (GB)
(72) Inventor: Deghenghi, Romano, Dr., 1264 St.Cergue (CH)
(74) Representative: Miles, John Stephen
(86) International application number: EP9605393
(87) International publication number: WO97022620

(56) References cited:
- EP-A- 0 018 072
- WO-A-91/18016
- WO-A-96/10040
- J. PEDIATRIC ENDOCRIN. & METABOL., vol. 8, 1995, pages 311-313, XP000651785 DEGHENGHI R. ET AL.: "Small Peptides as Potent Releasers of Growth Hormone"
- LIFE SCIENCES, vol. 54, no. 18, 1994, pages 1321-1328, XP000651534 DEHENGHI R. ET AL.: "GH-REleasing Activity of Hexarelin, a new Growth Hormone Releasing Peptide, in Infant and Rats "

## Description

The present invention relates to oligopeptide compounds containing a D-2-alkyltryptophan amino acid and having and which are capable of releasing growth hormone (GH) from somatotropes, and are active by oral route.

### Background of the invention

The increase of growth hormone (GH) levels in mammals after the administration of compounds inducing GH release can yield to growth acceleration and muscular mass increase and enhanced production of milk, if sufficiently high GH levels are obtained after the administration. Moreover, it is known that the increase of growth hormone levels in mammals can be achieved by administering known growth hormone release agents, such as growth hormone release hormones (GHRH).

The increase of growth hormone levels in mammals can also be obtained by administering growth hormone release peptides, some of them having previously been described, for example in US 4,223,019, US 4,223,020, US 4,223,021, US 4,224,316, US 4,226,857, US 4,228,155, US 4,228,156, US 4,228,157, US 4,228,158, US 4,410,512, US 4,410,513, US 4,411,890 and US 4,839,344.

Therefore, rather simple short chain-oligopeptides, capable of promoting growth hormone release on condition that they are easily and conveniently preparable, as well as of easy purification and formulation and active when administered by the oral route, are presently desired. Deghenghi et al. (Journal of Pediatric Endocrinology & Metabolism, 8, 311-313 (1995) disclose tetrapeptide aminoacyl derivatives which are active in vivo in releasing growth hormone. WO96/10040 discloses tetrapeptides containing D-2-alkyltryptophan capable of promoting the release of growth hormone by oral route. Deghenghi et al (Life Sciences, Vol 54. No.18, 1321-1328, 1994) discuss the GH-releasing activity of the hexapeptide Hexarelin.

### Summary of the invention

In a completely surprising manner it has now been found that very short oligopeptides, having at least one D-2-alkyltryptophan (2-Mrp) residue, have activity releasing growth hormone (GH) from somatotropes.

Another unexpected distinctive feature of the present invention is the very high potency and the favourable oral activity oral/potency ratio that even the smallest tripeptides of the series exhibit.

The oligopeptides of the present invention have the formula:

A-D-X-D-Mrp-B

wherein A is hydrogen, 2-aminoisobutyryl, 4-aminobutyryl, D relates to the dextro isomer, X is Mrp, wherein Mrp means 2-alkyltryptophan of formula: 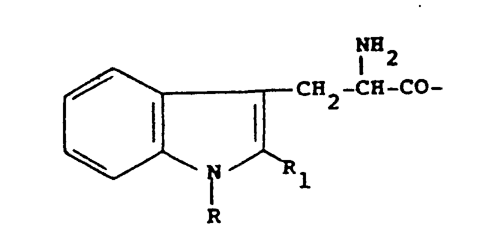 wherein R is hydrogen, CHO, SO₂CH₃, mesitylene-2-sulfonyl, PO₃(CH₃)₂, PO₃H₂; R₁ is a C₁-C₃ alkyl group; or
X is a residue of protected serine, Ser (Y), wherein Y can be benzyl, p-chlorobenzyl, 4-methoxybenzyl, 2,4,6-trimethoxybenzyl, tert-butyl; B is NR₂R₃, wherein R₂ and R₃, which can be the same or different, are hydrogen or a C₁-C₃ alkyl group; a OR₄ group, wherein R₄ is hydrogen or a C₁-C₃ alkyl; or B is C-Lys-NH₂ group, wherein C is Phe or Mrp.

### Detailed Disclosure of the Invention

The present invention lies on the discovery that different short-chain oligopeptides which promote the release and increase of growth hormone levels in blood of animals are characterized in that all of them comprise in the peptide chain a D-isomer of 2-alkyltryptophan (D-2-Me-Trp or D-Mrp).

The oligopeptides comprised in the scope of the present invention are defined by the following formula

A-D-X-D-Mrp-B

wherein A is hydrogen, 2-aminoisobutyryl, 4-aminobutyryl, D relates to the dextro isomer, X is Mrp, wherein Mrp means 2-alkyltryptophan of formula: 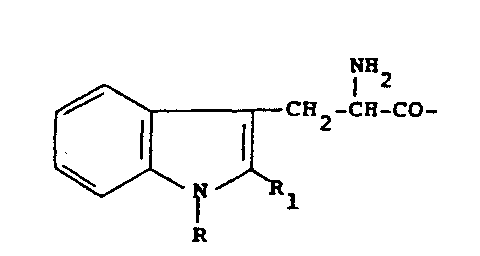 wherein R is hydrogen, CHO, SO₂CH₃, mesitylene-2-sulfonyl, PO₃(CH₃)₂, PO₃H₂; R₁ is a C₁-C₃ alkyl group; or
X is a residue of protected serine, Ser (Y), wherein Y can be benzyl, p-chlorobenzyl, 4-methoxybenzyl, 2,4,6-trimethoxybenzyl, tert-butyl; B is NR₂R₃, wherein R₂ and R₃, which can be the same or different, are hydrogen or a C₁-C₃ alkyl group; a OR₄ group, wherein R₄ is hydrogen or a C₁-C₃; or B is alkyl C-Lys-NH₂ group, wherein C is Phe or Mrp, and the addition salts with pharmaceutically acceptable organic or inorganic acids of any one of said polipeptides.

The abbreviations for the residues of amino acids herein used are in agreement with the standard nomenclature for the peptides:
Lys = L-Lysine.
Moreover,
Aib = 2-aminoisobutyryl;
GAB = 4-aminobutyryl;
Mrp = 2-alkyltryptophan;
Bzl = benzyl;
p-Cl-Bzl = p-chlorobenzyl;
Mob = 4-methoxybenzyl;
Tmob = 2,4,6-trimethoxybenzyl;
tbu = tert-butyl;
For = formyl;
Mts = mesitylene-2-sulfonyl.

According to the present invention, alkyl means lower alkyl, comprising from 1 to 3 carbon atoms. Examples of lower alkyl are methyl, ethyl, propyl, isopropyl. Among these, the methyl group is most preferred.

All the three letter-abbreviations of the amino acids preceded by a "D" indicate the D-configuration of the amino acid residue. When the amino acid is referred to with the only three-letter abbreviation, it has L configuration.

The preferred growth hormone-release compounds of the present invention are:
(a) GAB-D-Mrp-D-Mrp-Phe-Lys-NH₂;
(b) GAB-D-Mrp-D-Mrp-Mrp-Lys-NH₂;
(c) Aib-D-Mrp-D-Mrp-NH₂;
(d) Aib-D-Mrp-Mrp-NH₂;
(e) Aib-D-Ser(Bzl)-D-Mrp-NH₂;
wherein Mrp is 2-methyltryptophan, GAB and Aib are as defined above, and the addition salts with pharmaceutically acceptable organic or inorganic acids of anyone of said oligopeptides.

These compounds are preferably administered by the oral route, but they also can be administered intranasally or parenterally, or they can be formulated in controlled release systems, such as biodegradable microcapsules, microspheres, subcutaneous implants and the like.

The oligopeptide compounds according to the present invention can be synthesized according to the usual methods of peptide chemistry, both solid-phase and solution, or by means of the classical methods known in the art. The solid-phase synthesis starts from C-terminal end of peptide. A suitable starting material can be prepared, for example attaching the required protected alpha-amino acid to a chloromethylated resin, a hydroxymethylated resin, a benzhydrylamine resin (BHA), or to a para-methylbenzhydrylamine resin (p-Me-BHA). As example, a chloromethylated resin is sold with the Trade Mark BIOBEADS (R) SX 1 by BioRad Laboratories, Richmond, California. The preparation of the hydroxymethyl resin is described by Bodansky et al., Chem. Ind. (London) 38, 15997, (1966). The BHA resin is described by Pietta and Marshall, Chem. Comm., 650 (1970) and is commercially available by Peninsula Laboratories Inc., Belmont, California.

After the starting attachment, the alpha-amino acid-protecting group can be removed by means of different acid reagents, comprising trifluoroacetic acid (TFA) or hydrochloric acid (HCl) dissolved in organic solvents at room temperature. After the removal of the alpha-amino acid-protecting group, the remaining protected amino acids can be coupled step by step in the desired order. Each protected amino acid can generally be reacted in excess of about three times using a suitable carboxyl activating group, such as dicyclohexylcarbodiimide (DCC) or diisopropylcarbodiimide (DIC) dissolved, for example, in methylene chloride (CH₂Cl₂) or dimethylformamide (DMF) and their mixtures. After the desired amino acid sequence has been completed, the desired peptide can be cleaved from the supporting resin by treatment with a reagent such as hydrogen fluoride (HF), which not only cleaves the peptide from the resin, but also the more common protecting groups of the lateral chains. When a chloromethylated resin or a hydroxymethylated resin is used, the treatment with HF leads to the formation of the acid peptide in free form. When a BHA or p-Me-BHA resin is used, the treatment with HF directly leads to the formation of the amide peptide in free form.

The above discussed solid-phase procedure is known in the art and was described by Atherton and Sheppard, Solid Phase Peptide Synthesis (IRL Press, Oxford, 1989).

Some methods in solution, which can be used to synthesize the peptide moieties of the present invention are detailed in Bodansky et al., Peptide Synthesis, 2^{nd} edition, John Wiley & Sons, New York, N.Y. 1976 and from Jones, The Chemical Synthesis of Peptides, (Clarendon Press, Oxford, 1994).

These compounds can be administered to animals and humans at an effective dose which can be easily determined by the expert in the field and which can vary according to the specie, age, sex and weight of the treated subject. For example, in humans, when intravenously administered, the preferred dose falls in the range from about 0.1 µg to 10 µg of total peptide per kg of body weight. When orally administered, typically higher amounts are necessary. For example, in humans for the oral administration, the dosage level is typically from about 30 µg to about 1000 µg of polypeptide per kg of body weight. The exact level can be easily determined empirically based on the above disclosure.

Compositions, which comprise as active ingredient the organic and inorganic addition salts of the above described oligopeptides and their combinations, optionally, in admixture with a vehicle, diluent, matrix or delayed release coating, are also comprised in the scope of the present invention. The delayed release pharmaceutical forms, comprising bioerodible matrixes, suitable for subcutaneous implant are particularly interesting. Examples of these matrices are described in WO9222600 and W09512629.

### Biological activity

In vivo activity of these compounds was determined in ten day-rats, which were subcutaneously injected (s.c.) with a dose of 300 µg/kg or with different doses in dose-response studies, according to what described in detail by R. Deghenghi et. al, Life Sciences 54, 1321, (1994). The results are resumed in the Table below, the released GH was measured after 15 minutes from the treatment.

**TABLE**

| Peptide of example | Dose µg/kg s.c. | released GH (ng/ml) |
|---|---|---|
| 1 | 300 | 155.4 ∓ 19.7 |
| 2 | 300 | 165.4 ∓ 18.5 |
| 3 | 300 | 174.2 ∓ 25.9 |
| 4 | 300 | 64.2 ∓ 12.6 |
| 5 | 1.2 mg/kg | 59.4 ∓ 12.3 |
| Controls | - | 7 - 23 |

The following examples further illustrate the invention:

### Example 1

Making use of the solid-phase peptide synthesis technique as described in "Solid phase peptide synthesis" by E.Atherton and R.C. Sheppard, IRL Press, Oxford University Press, 1984, using fluorenylmethoxycarbonyl (Fmoc) as the protecting group, the peptide:

GAB-D-2-Mrp-D-2-Mrp-Phe-Lys-NH₂,

was prepared, wherein Mrp is 2-methyltryptophan, M.W. 779.9, found 778.4; purity (HPLC) 98.0%.

### Example 2

Analogously to Example 1, the following peptide was prepared:

GAB-D-2-Mrp-D-2-Mrp-2-Mrp-Lys-NH₂,

wherein Mrp is 2-methyltryptophan, M.W. 830.8, found 831.3; purity (HPLC) 98.0%.

### Example 3

Analogously to Example 1, the following peptide was prepared:

Aib-D-2-Mrp-D-2-Mrp-NH₂,

wherein Mrp is 2-methyltryptophan, M.W. 502.6, found 503.3; purity (HPLC) 99.0%.

### Example 4

Analogously to Example 1, the following peptide was prepared:

Aib-D-2-Mrp-2-Mrp-NH_{2.}

wherein Mrp is 2-methyltryptophan, M.W. 502.6, found 503.3; purity (HPLC) 99.0%.

### Example 5

Analogously to Example 1, the following peptide was prepared:

Aib-D-Ser(Bzl)-D-Mrp-NH₂.

wherein Mrp is 2-methyltryptophan, M.W. 479.6, found 480.5; purity (HPLC) 99.0%.

## Claims

1. A peptide of formula:
A-D-X-D-Mrp-B
wherein A is hydrogen, 2-aminoisobutyryl, 4-aminobutyryl, D relates to dextro isomer, X is Mrp, wherein Mrp means 2-alkyltryptophan of formula: 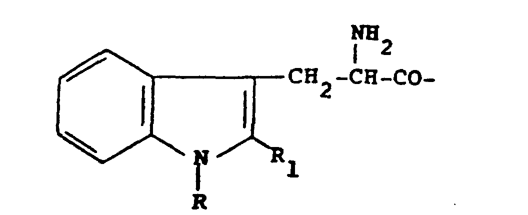 wherein R is hydrogen, CHO, SO₂CH₃, mesitylene-2-sulfonyl, PO₃(CH₃)₂, PO₃H₂; R₁ is a C₁-C₃ alkyl group; or
X is a residue of protected serine, Ser (Y), wherein Y can be benzyl, p-chlorobenzyl, 4-methoxybenzyl, 2,4,6-trimethoxybenzyl, tert-butyl; B is NR₂R₃, wherein R₂ and R₃, which can be the same or different, are hydrogen or a C₁-C₃ alkyl group; a OR₄ group, wherein R₄ is hydrogen or a C₁-C₃; or B is alkyl C-Lys-NH₂ group, wherein C is Phe or Mrp,
and the addition salts with pharmaceutically acceptable organic or inorganic acids of anyone of said polipeptides.

2. The peptide according to claim 1, wherein Mrp is selected from 2-methyltryptophan, 2-ethyltryptophan, 2-propyltryptophan, 2-isopropyltryptophan.

3. A peptide according to claim 1, wherein Mrp is 2-methyltryptophan.

4. The peptide according to claim 1, wherein A is 2-aminoisobutyryl, 4-aminobutyryl.

5. The peptide according to claim 1, wherein B is C-LysNH₂ wherein C is as defined above.

6. The peptide according to claim 1, having formula:
GAB-D-Mrp-D-Mrp-Phe-Lys-NH₂.

7. The peptide according to claim 1, having formula:
GAB-D-Mrp-D-Mrp-Mrp-Lys-NH₂.

8. The peptide according to claim 1, having formula:
Aib-D-Mrp-D-Mrp-NH₂.

9. The peptide according to claim 1, having formula:
Aib-D-Mrp-Mrp-NH₂.

10. The peptide according to claim 1, having formula:
Aib-D-Ser(Bzl)-D-Mrp-NH₂.

11. A peptide according to claims 6-10, wherein Mrp is 2-methyltryptophan.

12. The use of the peptides of claims 1-11 for the manufacturing of a medicament useful for promoting the release of growth hormone in an animal.

13. The use according to claim 12, wherein the medicament is useful in human medicine.

14. Pharmaceutical compositions comprising an effective amount of at least one peptide of claims 1-11 as active ingredient, optionally in admixture with carriers and excipients.

15. Pharmaceutical compositions according to claim 14 in the form of compositions for parenteral, intranasal, oral, controlled release administrations, subcutaneous implants.

16. Compositions according to claim 14 in the form of compositions for the oral administration.

## Patentansprüche

1. Peptid der Formel:
A-D-X-D-Mrp-B
worin A Wasserstoff, 2-Aminoisobutyryl, 4-Aminobutyryl ist, D sich auf das Dextrolsomer bezieht, X Mrp ist, worin Mrp ein 2-Alkyltryptophan der Formel: 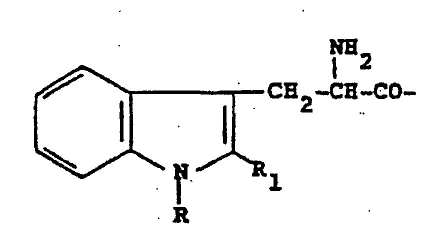 ist, worin R Wasserstoff, CHO, SO₂CH₃, Mesitylen-2-sulfonyl, PO₃(CH₃)₂, PO₃H₂ ist; R₁ eine (C₁-C₃)Alkylgruppe ist; oder
X ein Rest eines geschütztes Serins, Ser (Y), ist, worin Y Benzyl, p-Chlorbenzyl, 4-Methoxybenzyl, 2,4,6-Trimethoxybenzyl, tert-Butyl sein kann; B NR₂R₃, worin R₂ und R₃ gleich oder verschieden sein können und Wasserstoff oder eine (C₁-C₃)Alkylgruppe sind; eine OR₄-Gruppe ist, worin R₄ Wasserstoff oder (C₁-C₃)Alkyl ist; oder B eine C-Lys-NH₂-Gruppe ist, worin C Phe oder Mrp ist,
und Additionssalze mit pharmazeutisch verträglichen organischen oder anorganischen Säuren irgendeines der genannten Polypeptide.

2. Peptid nach Anspruch 1, worin Mrp ausgewählt wird aus 2-Methyltryptophan, 2-Ethyltryptophan, 2-Propyltryptophan, 2-Isopropyltryptophan.

3. Peptid nach Anspruch 1, worin Mrp 2-Methyltryptophan ist.

4. Peptid nach Anspruch 1, worin A 2-Amlnoisobutyryl, 4-Aminobutyryl ist.

5. Peptid nach Anspruch 1, worin B C-Lys-NH₂ Ist, worin C wie oben definiert ist.

6. Peptid nach Anspruch 1, mit der Formel:
GAB-D-Mrp-D-Mrp-Phe-Lys-NH₂,

7. Peptid nach Anspruch 1, mit der Formel:
GAB-D-Mrp-D-Mrp-Mrp-Lys-NH₂.

8. Peptid nach Anspruch 1, mit der Formel:
Aib-D-Mrp-D-Mrp-NH₂.

9. Peptid nach Anspruch 1, mit der Formel:
Aib-D-Mrp-Mrp-NH₂.

10. Peptid nach Anspruch 1, mit der Formel:
Aib-D-Ser(Bzl)-D-Mrp-NH₂.

11. Peptid nach Ansprüchen 6 bis 10, worin Mrp 2-Methyltryptophan ist.

12. Verwendung der Peptide nach den Ansprüchen 1 bis 11 für die Herstellung eines Medikamentes, das nützlich zur Förderung der Freisetzung von Wachstumshormonen in Tieren ist.

13. Verwendung noch Anspruch 12, worin das Medikament nützlich In der Humanmedizin ist.

14. Pharmazeutische Zusammensetzung, die eine wirksame Menge mindestens eines Peptides nach Ansprüchen 1 bis 11 als aktiven Bestandtell wahlwelse in Zusammenmischung mit Trägern und Exzlpienten umfasst.

15. Pharmazeutische Zusammensetzung nach Anspruch 14 in der Form von Zusammensetzungen für die parenterale, intranasale, orale, mit kontrollierter Freisetzung erfolgende Verabrelchung, und für subkutane Implantate.

16. Zusammensetzung nach Anspruch 14 In der Form von Zusammensetzungen für die orale Verabreichung.

## Revendications

1. Peptide de formule :
A-D-X-D-Mrp-B
dans laquelle A est un hydrogène, un groupe 2-aminoisobutyryle, 4-aminobutyryle, D désigne l'isomère dextro, X est Mrp, où Mrp représente un groupe 2-alkyltryptophane de formule : 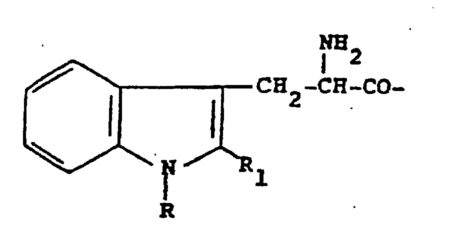 dans laquelle R est de l'hydrogène, CHO, SO₂CH₃, un groupe mésitylène-2-sulfonyle, PO₃(CH₃)₂, PO₃H₂ ; R₁ est un groupe alkyle en C₁ à C₃ ; ou X est un résidu de sérine protégée, Ser(Y), où Y peut être un groupe benzyle, p-chlorobenzyle, 4-méthoxybenzyle, 2,4,6-triméthoxybenzyle, tert-butyle ; B est NR₂R₃, où R₂ et R₃, qui peuvent être identiques ou différents, sont de l'hydrogène ou un groupe alkyle en C₁ à C₃ ; un groupé OR₄, où R₄ est de l'hydrogène ou un groupe en C₁ à C₃ ; où B est un groupe alkyle C-Lys-NH₂, où C est Phe ou Mrp,
et les sels d'addition avec des acides organiques ou minéraux pharmaceutiquement acceptables de l'un quelconque desdits polypeptides.

2. Peptide selon la revendication 1, dans lequel Mrp est choisi parmi le 2-méthyltryptophane, le 2-éthyltryptophane, le 2-propyltryptophane, le 2-isopropyltryptophane.

3. Peptide selon la revendication 1, dans lequel Mrp est le 2-méthyltryptophane.

4. Peptide selon la revendication 1, dans lequel A est un groupe 2-aminoisobutyryle, 4-aminobutyryle.

5. Peptide selon la revendication 1, dans lequel B est un groupe C-LysNH₂ dans lequel C est tel que défini ci-dessus.

6. Peptide selon la revendication 1, ayant la formule :
GAB-D-Mrp-D-Mrp-Phe-Lys-NH₂.

7. Peptide selon la revendication 1, ayant la formule :
GAB-D-Mrp-D-Mrp-Mrp-Lys-NH₂.

8. Peptide selon la revendication 1, ayant la formule :
Aib-D-Mrp-D-Mrp-NH₂.

9. Peptide selon la revendication 1, ayant la formule :
Aib-D-Mrp-Mrp-NH₂.

10. Peptide selon la revendication 1, ayant la formule :
Aib-D-Ser(Bzl)-D-Mrp-NH₂.

11. Peptide selon les revendications 6 à 10, dans lequel Mrp est le 2-méthyltryptophane.

12. Utilisation des peptides selon les revendications 1 à 11 pour la fabrication d'un médicament utile pour activer la libération de l'hormone de croissance chez un animal.

13. Utilisation selon la revendication 12, dans laquelle le médicament est utile en médecine humaine.

14. Compositions pharmaceutiques comprenant une quantité efficace d'au moins un peptide selon les revendications 1 à 11 en tant qu'ingrédient actif, éventuellement mélangé avec des véhicules et des excipients.

15. Compositions pharmaceutiques selon la revendication 14 sous la forme de compositions pour administrations parentérale, intranasale, orale, à libération contrôlée, pour des implants sous-cutanés.

16. Compositions selon la revendication 14, sous la forme de compositions pour l'administration orale.
